(19) Europäisches Patentamt

European Patent Office

Office européen des brevets

(11) Publication number: **0 200 390 B1**

## EUROPEAN PATENT SPECIFICATION

(45) Date of publication of patent specification: **10.03.93**  (51) Int. Cl.5: **A61B  17/36**

(21) Application number: **86302603.5**

(22) Date of filing: **08.04.86**

(54) **Ablation of atherosclerotic plaque from a patient.**

(30) Priority: **08.04.85 US 720595**

(43) Date of publication of application:
**05.11.86 Bulletin  86/45**

(45) Publication of the grant of the patent:
**10.03.93 Bulletin  93/10**

(84) Designated Contracting States:
**AT BE CH DE FR GB IT LI LU NL SE**

(56) References cited:
**EP-A- 0 172 490**
**EP-A- 0 194 856**
**WO-A-86/02783**
**GB-A- 2 125 986**

**R. MARKS et al.: "Bioengineering and the skin", July 1979, pages 253-265, MTP Press Ltd., Falcon House, Lancaster, GB; R.R. AN-DERSON et al.: "Optical radiation transfer in the human skin and applications in in vivo remittance spectroscopy"**

**J.H. Walter "Wirkungsmechanismen von Laserstrahlung im biologischen Gewebe" in Berlin.Müller "Angewandte Lasermedizin", 3. Ergänzungslieferung,11/90, II-31, pages 1-5;**

(73) Proprietor: **THE GENERAL HOSPITAL CORPO-RATION**
**55 Fruit Street**
**Boston, MA 02114(US)**

(72) Inventor: **Prince, Martin**
**2-1402 Peabody Terrace**
**Cambridge Massachusetts 02138(US)**
Inventor: **Oseroff, Allan**
**14 Jefferson Road**
**Brookline Massachusetts 02146(US)**
Inventor: **Parrish, John A.**
**11 Newton Street**
**Weston Massachusetts 02193(US)**

(74) Representative: **Deans, Michael John Percy**
**Lloyd Wise, Tregear & CO. Norman House**
**105-109 Strand**
**London WC2R OAE (GB)**

J.R. Spears et al. "Fluorescence of experimental atheromatous plaques with hematoporphyrin derivative", J. Clin.Invest, Vol.71, Feb. 1983, pages 395-399;

Anderson,Parrish "Selective Photothermolysis: Precise microsurgery by selective absorption of pulsed radiation", Science, Vol.220, April 1983, pages 524-527;

## Description

This invention relates to the ablation of atherosclerotic plaque in a patient.

Ablation of atherosclerotic plaque has previously been attempted in patients using a variety of lasers, e.g. argon lasers, at various wavelengths, e.g. 514 nm (for argon lasers). The technique typically involves the use of a flexible optical fibre associated with the laser, as described, e.g. in Choy U.S. Pat. No. 4,207,874. In such known techniques, the plaque is first visualized, either by angiography or using a coherent fibre optic bundle built into the laser catheter, and the ablating laser is then aimed and "fired" at the plaque.

There have been some attempts to improve selectivity in the absorption of laser light by plaques, compared to surrounding tissues, by selectively staining the plaque with, e.g. hematoporphyrin (Spears et al 1983) J. Clin Invest. 71, 395-399). Hematoporphyrin is a photosensitizing agent which, when exposed to laser light, may bring about the photochemical destruction of the plaque.

GB-A-2125986 discloses a device for the treatment of cancers comprising a pulsed laser beam source, an image detecting means and an endoscope which is arranged to transmit the pulsed laser beam from the pulsed laser beam source and to direct the beam onto a cancer focus to sterilize the cancerous cells. The endoscope also serves to transmit light from the cancer focus and the surface of the affected organism to the image detecting means to enable diagnosis and observation of the cancer focus to be made.

EP-A-0194856 discloses an apparatus and method for irradiating a treatment area within a body. The method includes introducing elongated flexible radiation transfer conduit (fiberoptic arrays) into the body cavity with the distal end thereof operatively opposite the treatment area. At the other end of the conduit a particular optical characteristic of the treatment area (such as fluorescence) is photoelectrically sensed and as long as the particular optical characteristic is sensed there is periodically transmitted laser pulses via the conduit to the treatment site.

In a first aspect of this invention, we provide, in combination: a carotenoid to be selectively taken up by atherosclerotic plaque in a patient, and a laser apparatus which provides pulsed laser light with a wavelength at which the ratio of Kubelka-Munk absorption coefficients of said plaque substance to normal aortic endothelium is at least 1.5:1, and preferably at least 2:1, with a pulse duration less than the thermal relaxation time of the volume of the tissue defined by the diameter of the laser beam within the tissue and the depth at which 67% of the incident light of said laser has been absorbed, and at a beam intensity sufficiently high to cause heating of the target plaque, at the said wavelength and with the said pulse duration, to a temperature effective to burn away a substantial portion of said plaque but not to cause unacceptable damage to surrounding healthy tissue.

In a second and alternative aspect of this invention, we provide, in combination : a supply of a foodstuff containing a carotenoid to be selectively taken up, upon ingestion by a patient, by atherosclerotic plaque in said patient, and a laser apparatus which provides pulsed laser light with a wavelength at which the ratio of Kubelka-Munk absorption coefficients of said plaque to normal aortic endothelium is at least 1.5:1, and preferably at least 2:1, with a pulse duration less than the thermal relaxation time of the volume of the tissue defined by the diameter of the laser beam within the tissue and the depth at which 67% of the incident light of said laser has been absorbed, and at a beam intensity sufficiently high to cause heating of the target plaque, at the said wavelength and with the said pulse duration, to a temperature effective to burn away a substantial portion of said plaque but not to cause unacceptable damage to surrounding healthy tissue.

In preferred arrangements, the carotenoid is administered orally or intravenously; the administered carotenoid is $\beta$-carotene; the wavelength of the laser light as 440-480 nm, more preferably about 460 nm; and administration of $\beta$-carotene is oral and is carried out at least once daily for at least two days, and more preferably at least one week, prior the exposure to the laser light, at a dosage of between 100 mg and 5,000 mg per day, and preferably at least 300 mg per day.

In a third and further alternative aspect of the invention, we provide use of a carotenoid for the manufacture of a medicament for administration to a patient in treatment to ablate atherosclerotic plaque in which treatment, following such administration, the plaque is exposed to pulsed laser light having a wavelength at which the ratio of Kubelka-Munk absorption coefficients of said plaque to normal aortic endothelium is at least 1.5:1, and preferably at least 2:1, a pulse duration less than the thermal relaxation time of the volume of the tissue defined by the diameter of the laser beam within the tissue and the depth at which 67% of the incident light of said laser has been absorbed, and at a beam intensity sufficiently high to cause heating of the target plaque at the said wavelength and with the said pulse duration, to a temperature effective to burn away a substantial portion of said plaque but not to cause unacceptable damage to surrounding healthy tissue.

In preferred arrangements the carotenoid is β-carotene. A foodstuff containing the carotenoid may be used.

The invention is hereinafter more particularly described by way of example only with reference to the accompanying drawings.

The drawings will first be described.

Drawings

Fig. 1 is a diagrammatic representation of apparatus used to determine the wavelength of tissue absorption.

Fig. 2 is a diagrammatic representation of a laser and associated apparatus used to demonstrate selective plaque ablation according to the invention.

Fig. 3 is a graph showing ratios of absorption coefficients for normal human aortic endothelium and aortic plaque over a range of wavelengths.

Absorption and Wavelength

The first step in the plaque ablation method described is to select a wavelength at which the plaque:normal aorta Kubelka-Munk absorption coefficent is at least 1.5:1. This difference in absorption coefficients (seen at about 460 nm in patients not administered supplemental chromophore) results from the preferential accumulation in plaque of dietary carotenoids.

Where a chromophore is administered to the patient prior to ablation, the wavelength employed will reflect the concommitant change in the composition of the plaque. For example, administration of a chromophore, e.g., capsanthin, which absorbs at a wavelength above 460 nm will bring about some increase in the optimal wavelength. However, because carotenoids are already present in the plaque, the administration of a chromophore absorbing above or below the pre-administration optimum will generally result in an optimal wavelength somewhere between the absorption maximum of the administered chromophore, and the pre-administration optimum. The absorption maxima of ten suitable carotenoids known to exist in humans and, in some instances, in foods, are given in the table below.

| carotenoid | absorption maxima in light petroleum (nm) |
|---|---|
| lycopene | 446,472,505 |
| beta-carotene | 425,451,480 |
| canthaxanthin | 466 |
| alpha carotene | 422,444,480 |
| zeta carotene | 378,400,425 |
| lutein | 420,447,477 |
| cryptoxanthin | 425,451,483 |
| zeaxanthin | 423,451,483 |
| capsanthin | 474-475,504 |
| capsorubin | 444,474,506 |

The Kubelka-Munk absorbance coefficients of plaque and normal aortic tissue are measured by conventional techniques, as is explained in more detail below.

Chromophore Administration

The amount, duration, and mode of administration of the chromophore will depend on its properties, e.g., toxicity and affinity for plaque. Some carotenoids are naturally present in high concentrations in foods such as carrots and tomatoes, and administration of these could be achieved at least in part by daily ingestion of such foods. The chromophore can also be administered, orally, admixed with a pharmaceutically acceptable carrier substance, in a pill or liquid, or can be administered intravenously or intraperitoneally.

It is particularly useful to employ a chromophore which, in addition to being preferentially accumulated in plaque, fluoresces, facilitating detection and laser aiming. Carotenoids, for example, advantageously fluoresces when excited at 460 nm.

## Pulse Duration

As mentioned above, the pulse duration used is related to the thermal relaxation time t of the volume of tissue defined by the diameter of the laser beam within the tissue and the depth at which 67% of the incident light of the laser has been absorbed; the relaxation time is the time it takes for the temperature increase ($\Delta$ T) caused by a pulse of light of a duration approaching zero to decrease by one-half at the surface of that tissue volume after the pulse has ceased.

Thermal relaxation time t is roughly approximated by:

$$t = \frac{d^2}{2K}$$

where d, expressed in cm, is the smaller of the diameter of the irradiated volume (conventionally referred to as "D") and the depth at which 67% of the incident light has been absorbed (conventionally referred to as "d"). K is the thermal difussivity, in cm$^2$/sec.; K is approximately 0.0013, for water. It should be emphasized that this method of calculating t is given for convenience only; where reference is made herein to the relationship between pulse duration and t, actual t, as defined earlier in terms of a one-half decrease in $\Delta$ T, is intended.

## Pulse Fluence

The fluence (Joules/cm$^2$) of the laser light should be sufficiently high to cause heating of the target plaque, at the wavelength and pulse duration employed, to a temperature at which a substantial portion of the plaque is burned away, and not so high as to cause unacceptable damage to surrounding healthy tissue. The requisite fluence range varies with the diameter of the optical fiber used in conjunction with the laser. Generally, a fluence of 2-10J/cm$^2$ is preferred for a 1 mm diameter optical fibre, while smaller diameter fibres will require higher fluences to compensate for scattering losses at the periphery of the illustrated volume.

For fluorescence detection, a lower, non-burning intensity, e.g., about 1 milliwatt, is used in a pulsing or continuous mode to excite fluorescence.

## Laser and Associated Apparatus

Any laser which can deliver pulses of the desired intensity, duration, and wavelength can be used. A preferred laser is a flashlamp pumped pulse dye laser. The fibre optic bundles and auxiliary apparatus by which laser light is delivered to the plaques can be of any conventional configuration, e.g., as described in Choy, id. A second fibre optic bundle can be used for plaque detection, and if fluorescence of a plaque-associated carotenoid is to be employed in detection, excitation can be achieved by means of a third optic bundle, or by use of the ablation bundle in an excitation mode.

A particular plaque ablation procedure was carried out as follows.

## Kubelka-Munk Absorbance Coefficients

Absorbance coefficients were determined by measuring remittance and transmittance of normal aortic endothelium and aortic plaque, essentially by the integrating sphere technique described in Anderson et al. (1979) Proc. Symp. Bioengineer and the Skin, Cardiff, Wales (MTP Press, London).

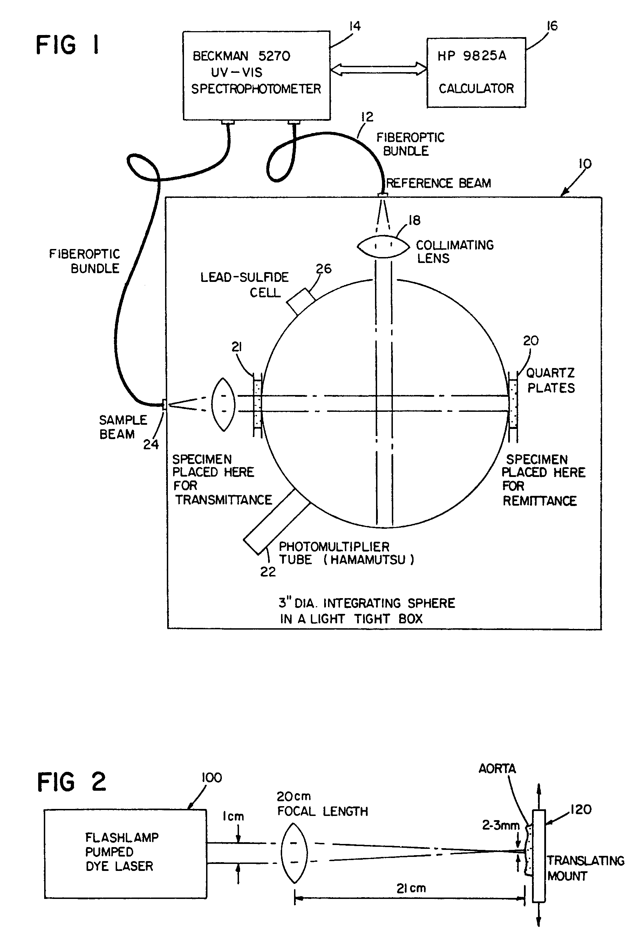

The procedure was carried out using the apparatus shown in Fig. 1, including 7.5 cm diameter barium sulphate coated integrating sphere 10, connected by 2 mm diameter quartz fibre optic bundles 12 to Beckman 5270 double beam spectrophotometer 14, which is interfaced with Hewlett Packard 9825A computer 16 for digital data acquisition and analysis. Freshly coated barium sulphate plates (not shown) were used as 100% remittance standards. The apparatus further included collimating lens 18, for providing a collimating light beam to illuminate a 5 mm diameter region of the tissue; quartz sample-holding plates 20 and 21; photomultiplier tube 22; beam source 24; fibre optic bundle 26, connecting beam source 24 with spectrophotometer 14; and lead sulphide cell 26.

To use the apparatus of Fig. 1 to determine absorption coefficients, cadaveric specimens of human aorta were obtained less than 48 hours post mortem. Optical measurements were made on soft, yellow, raised plaques in which the outer portion of the media and adventicia had been stripped by blunt dissection.

5

Specimens were .2 to 2 mm thick. The specimens were mounted sandwiched between polished quartz plates 20 (for remittance measurement) or 21 (for transmittance measurement), and the tissue surface irregularities filled in with saline to replace the irregular air-tissue interface with a smooth air-quartz interface. The air-quartz interface had a consistent and predictable remittance that was measured and subtracted from the tissue remittance measurement.

The tissue-quartz plate sandwiches were placed on the integrating sphere apparatus of Fig. 1; remittance and transmittance measured as in Anderson et al., id, and Kubelka-Murk absorption coefficients calculated as described therein.

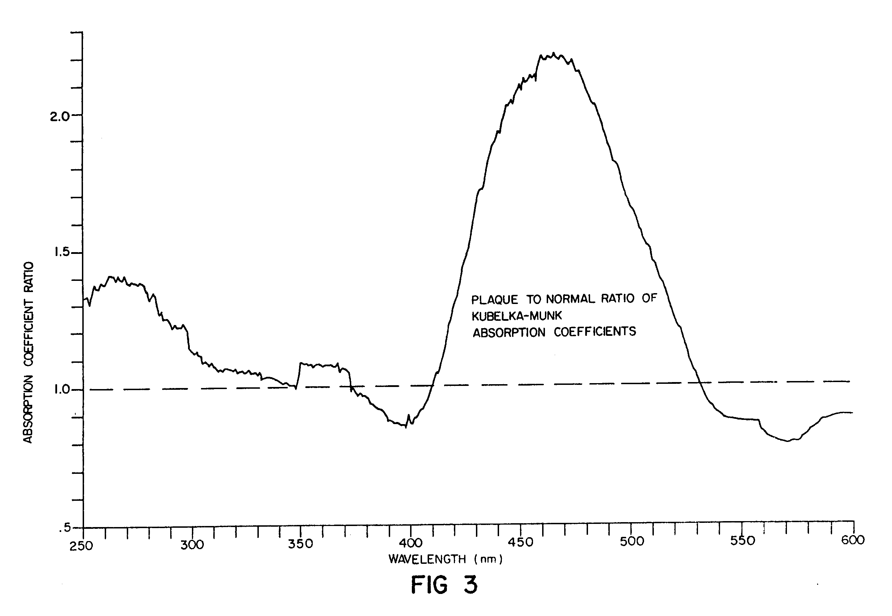

The results are given in Fig. 3, expressed as the plaque to normal ratio. As shown in Fig. 3, the ratio is highest between 440 and 480 nm, with a peak at 460 nm.

Plaque Ablation

Ablation of aortic plaques of cadavers was carried out using the apparatus of claim 2, including Candella SLL 500 Coaxial Flashlamp pumped dye laser 10, translating tissue mount 12, and a 40% output coupler (not shown).

The laser generated 1 microsecond pulses with energies up to 2 joules at 459 to 470 nm with coumarin 445 laser dye (Exciton # C445), $1.5 \times 10^{-4}$ molar in 50% methanol and 50% distilled water. Laser wavelength output was measured with a high intensity monochrometer (Bausch Lomb # 33-86-76) and the pulse width characterized with an ultrafast silicon photodiode (EG&G FND 100Q) reverse biased by 90 volts. The laser mirrors were adjusted until output burn patterns on polaroid film were circular. The 1 cm diameter light beam coming out of the laser was focused with a 20 cm focal length quartz lens to form a 2-3 mm diameter spot on the specimen. The specimen could then be translated across the laser beam to give an identical radiation dose to several normal and atheromatous regions of cadaver aorta, obtained as before. Specimens were irradiated, photographed fresh, fixed in formalin, bisected through the rows of irradiation sites, and photographed in cross section.

At 400 mJ per pulse, 20 pulses formed an obvious crater in plaque but caused only slight browning in, and no removal of, adjacent normal tissue. Plaque required 3.2 - 7.9 J/cm2 for removal and normal aortic tissue required 9.7 - 20.5 J/cm2. In general the yellower and softer atheromas had the lowest thresholds while hard and/or pale atheromas had higher threshold fluences.

## Claims

1. In combination: a carotenoid to be selectively taken up by atherosclerotic plaque in a patient, and a laser apparatus which provides pulsed laser light with a wavelength at which the ratio of Kubelka-Munk absorption coefficients of said plaque to normal aortic endothelium is at least 1.5:1, and preferably at least 2:1, with a pulse duration less than the thermal relaxation time of the volume of the tissue defined by the diameter of the laser beam within the tissue and the depth at which 67% of the incident light of said laser has been absorbed, and at a beam intensity sufficiently high to cause heating of the target plaque, at the said wavelength and with the said pulse duration, to a temperature effective to burn away a substantial portion of said plaque but not to cause unacceptable damage to surrounding healthy tissue.

2. In combination: a supply of a foodstuff containing carotenoid to be selectively taken up, upon ingestion by a patient, by atherosclerotic plaque in said patient, and a laser apparatus which provides pulsed laser light with a wavelength at which the ratio of Kubelka-Munk absorption coefficients of said plaque to normal aortic endothelium is at least 1.5:1, and preferably at least 2:1, with a pulse duration less than the thermal relaxation time of the volume of the tissue defined by the diameter of the laser beam within the tissue and the depth at which 67% of the incident light of said laser has been absorbed, and at a beam intensity sufficiently high to cause heating of the target plaque, at the said wavelength and with the said pulse duration, to a temperature effective to burn away a substantial portion of said plaque but not to cause unacceptable damage to surrounding healthy tissue.

3. The combination of Claim 1 or Claim 2, further characterised in that said carotenoid is $\beta$ -carotene.

4. The combination of Claim 1 or Claim 2, further characterised in that said wavelength is in the range of 440 to 480 nm., and preferably is about 460 nm.

5. The combination of Claim 1 or any claim appendant thereto, further characterised in that said carotenoid is provided in a dosage form enabling intravenous administration to a patient.

6. The combination of Claim 1 or Claims 3 or 4 as appendant to Claim 1, further characterised in that said carotenoid is provided in a dosage form enabling oral administration to a patient at least once daily for at least two days prior to exposure of said patient to said laser light.

7. The combination of Claim 1 or any claim appendant thereto, further characterised in that said carotenoid is provided in a dosage form enabling administration thereof to a patient at a dosage rate of between 100 mg and 5,000 mg per day.

8. Use of a carotenoid for the manufacture of a medicament for administration to a patient in treatment to ablate atherosclerotic plaque in which treatment, following such administration, the plaque is exposed to pulsed laser light having a wavelength at which the ratio of Kubelka-Munk absorption coefficients of said plaque to normal aortic endothelium is at least 1.5:1, and preferably at least 2:1, a pulse duration less than the thermal relaxation time of the volume of the tissue defined by the diameter of the laser beam within the tissue and the depth at which 67% of the incident light of said laser has been absorbed, and at a beam intensity sufficiently high to cause heating of the target plaque at the said wavelength and with the said pulse duration, to a temperature effective to burn away a substantial portion of said plaque but not to cause unacceptable damage to surrounding healthy tissue.

9. Use of $\beta$-carotene for the manufacture of a medicament for administration to a patient in a treatment as defined in Claim 8.

10. Use of a foodstuff containing a carotenoid for the manufacture of a medicament for administration to a patient in a treatment as defined in Claim 8.

11. Use of a raw foodstuff containing a carotenoid for the preparation of an agent for ingestion by a patient in a treatment to ablate atherosclerotic plaque in which treatment, following such ingestion, the plaque is exposed to pulsed laser light as defined in Claim 8.

**Patentansprüche**

1. In Kombination:
   - ein Carotenoid, das selektiv von atherosclerotischer Plaque in einem Patienten aufgenommen wird,
   - und eine Laser-Vorrichtung, die gepulstes Laserlicht mit einer Wellenlänge abgibt, bei der das Verhältnis der Kubelka-Munk-Absorptionskoeffizienten der genannten Plaque zu normalem Aorta-Endothel wenigstens 1,5 : 1, vorzugsweise wenigstens 2 : 1, beträgt,
   - mit einer Pulsdauer, die kleiner ist als die thermische Relaxationszeit des Gewebe-Volumens, das durch den Durchmesser des Laserstrahls innerhalb des Gewebes und der Tiefe, in der 67% des einfallenden Laserlichtes absorbiert worden ist, begrenzt (definiert) ist,
   - und mit einer Strahlintensität, die bei der genannten Wellenlänge und Pulsdauer genügend hoch ist, um die Target-Plaque auf eine Temperatur zu erwärmen, bei der ein wesentlicher Teil der Plaque weggebrannt wird, ohne daß ein unannehmbarer Schaden im umgegebenden gesunden Gewebe verursacht wird.

2. In Kombination:
   - eine Vorratsmenge an Nahrungsmittel, die (das) Carotenoid enthält, welches nach Einnahme durch einen Patienten selektiv von atherosclerotischer Plaque in dem Patienten aufgenommen wird,
   - und eine Laser-Vorrichtung, die gepulstes Laserlicht mit einer Wellenlänge abgibt, bei der das Verhältnis der Kubelka-Munk-Absorptionskoeffizienten der genannten Plaque zu normalem Aorta-Endothel wenigstens 1,5 : 1, vorzugsweise wenigstens 2 : 1, beträgt,
   - mit einer Pulsdauer, die kleiner ist als die thermische Relaxationszeit des Gewebe-Volumens, das durch den Durchmesser des Laserstrahls innerhalb des Gewebes und der Tiefe, in der 67% des einfallenden Laserlichtes absorbiert worden ist, begrenzt (definiert) ist,

7

- und mit einer Strahlintensität, die bei der genannten Wellenlänge und Pulsdauer genügend hoch ist, um die Target-Plaque auf eine Temperatur zu erwärmen, bei der ein wesentlicher Teil der Plaque weggebrannt wird, ohne daß ein unannehmbarer Schaden im umgegebenden gesunden Gewebe verursacht wird.

3. Kombination gemäß Anspruch 1 oder 2, dadurch gekennzeichnet, daß das genannte Carotenoid β-Carotin ist.

4. Kombination nach Anspruch 1 oder 2, dadurch gekennzeichnet, daß die genannte Wellenlänge im Bereich von 440 bis 480 nm liegt, vorzugsweise etwa 460 nm ist.

5. Kombination nach Anspruch 1 oder einem untergeordneten Anspruch, dadurch gekennzeichnet, daß das genannte Carotenoid in einer Dosierungsform vorgegeben wird, die eine intravenöse Verabreichung bei einem Patienten ermöglicht.

6. Kombination nach Anspruch 1 oder Anspruch 3 oder 4 als untergerordnet zu Anspruch 1, dadurch gekennzeichnet, daß das genannte Carotenoid in einer Dosierungsform vorgegeben wird, die eine orale Verabreichung bei einem Patienten ermöglicht, und zwar wenigstens einmal täglich für wenigstens zwei Tage, bevor der Patient dem genannten Laserlicht ausgesetzt wird.

7. Kombination nach Anspruch 1 oder einem diesen nachgeordneten Anspruch, dadurch gekennzeichnet, daß das genannte Carotenoid in einer Dosierungsfrom vorgegeben wird, die die Verabreichung bei einem Patienten mit einer Dosierungsrate zwischen 100 mg und 5.000 mg pro Tag ermöglicht.

8. Verwendung eines Carotenoids zur Herstellung eines Medikamentes, das einem Patienten zur Ablation (Ablösung) von atherosclerotischer Plaque verabreichbar ist, wobei nach der Verabreichung im Laufe dieser Ablations-Behandlung die Plaque gepulstem Laserlicht mit einer Wellenlänge ausgesetzt wird, bei der das Verhältnis der Kubelka-Munk-Absorptionskoeffizienten der genannten Plaque zu normalem Aorta-Endothel wenigstens 1,5 : 1, vorzugsweise wenigstens 2 : 1, beträgt, mit einer Pulsdauer, die kleiner ist als die thermische Relaxationszeit des Gewebe-Volumens, das durch den Durchmesser des Laserstrahls innerhalb des Gewebes und der Tiefe, in der 67% des einfallenden Laserlichtes absorbiert worden ist, begrenzt (definiert) ist, und mit einer Strahlintensität, die bei der genannten Wellenlänge und Pulsdauer genügend hoch ist, um die Target-Plaque auf eine Temperatur zu erwärmen, bei der ein wesentlicher Teil der Plaque weggebrannt wird, ohne daß ein unannehmbarer Schaden im umgegebenden gesunden Gewebe verursacht wird.

9. Verwendung von β-Carotin zur Herstellung eines Medikamentes, das einem Patienten bei einer Behandlung gemäß Anspruch 8 verabreichbar ist.

10. Verwendung eines Nahrungsmittels, das ein Carotenoid zur Herstellung eines Medikamentes enthält, das einem Patienten bei einer Behandlung gemäß Anspruch 8 verabreichbar ist.

11. Verwendung eines rohen Nahrungsmittels, das ein Carotenoid für die Präparation eines Agens zur Einnahme durch einen Patienten bei einer Behandlung zur Ablation (Ablösung) atherosklerotischer Plaque enthält, wobei bei dieser Behandlung, der Einnahme folgend, die Plaque einem gepulsten Laserlicht gemäß Anspruch 8 ausgesetzt wird.

**Revendications**

1. En combinaison : un caroténoïde qui doit être absorbé de façon sélective par la plaque athérosclérotique d'un patient, et un appareil laser émettant un rayon lumineux de longueur d'onde telle que le rapport des coefficients d'absorption de Kubelka-Munk de ladite plaque à un endothélium aortique normal est d'au moins 1,5:1, et de préférence au moins 2:1, la durée d'émission étant inférieure au temps de relaxation thermique du volume de tissu défini par le diamètre du rayon laser sur le tissu et une profondeur à laquelle 67% de la lumière incidente dudit laser sont absorbés, l'intensité du rayon étant suffisamment forte pour, par le choix d'une longueur d'onde et d'une durée d'émission définies, entraîner l'échauffement de la plaque ciblée à une température suffisante pour éliminer par brûlage une partie importante de ladite plaque sans causer de dommage inacceptable aux tissus sains environnants.

2.  En combinaison : une préparation alimentaire contenant un caroténoïde devant être, après ingestion par le patient, absorbé sélectivement par la plaque athérosclérotique dudit patient, et un appareil laser émettant un rayon lumineux de longueur d'onde telle que le rapport des coefficients d'absorption de Kubelka-Munk de ladite plaque à un endothélium aortique normal est d'au moins 1,5:1, et de préférence au moins 2:1, la durée d'émission étant inférieure au temps de relaxation thermique du volume de tissu défini par le diamètre du rayon laser sur le tissu et une profondeur à laquelle 67% de la lumière incidente dudit laser sont absorbés, l'intensité du rayon étant suffisamment forte pour, par le choix d'une longueur d'onde et d'une durée d'émission définies, entraîner l'échauffement de la plaque ciblée à une température suffisante pour éliminer par brûlage une partie importante de ladite plaque sans causer de dommage inacceptable aux tissus sains environnants.

3.  Combinaison selon la Revendication 1 ou la Revendication 2, caractérisée en outre en ce que ledit caroténoïde est du $\beta$-carotène.

4.  Combinaison selon la Revendication 1 ou la Revendication 2, caractérisée en outre en ce que ladite longueur d'onde se situe dans une plage allant de 440 à 480 nm., et de préférence aux environs de 460 nm.

5.  Combinaison selon la Revendication 1 ou l'une quelconque des revendications qui lui sont rattachées, caractérisée en ce que ledit caroténoïde est préparé sous une forme dont le dosage permet une administration intraveineuse au patient.

6.  Combinaison selon la Revendication 1 ou les Revendications 3 ou 4 lui étant rattachées, caractérisée en outre en ce que ledit caroténoïde est préparé sous une forme dont le dosage permet une administration orale au patient, au moins une fois par jour durant les deux jours précédant son exposition à la lumière laser.

7.  Combinaison selon la Revendication 1 ou l'une quelconque des Revendications lui étant rattachée, caractérisée en outre en ce que ledit caroténoïde est préparé sous une forme permettant une administration au patient d'une dose comprise entre 100 mg et 5,000 mg par jour.

8.  Utilisation d'un caroténoïde pour la fabrication d'un médicament devant être administré à un patient dans le traitement visant l'ablation de sa plaque athérosclérotique, traitement au cours duquel, après une telle administration, la plaque est exposée à une lumière laser émise à une longueur d'onde telle que le rapport des coefficients d'absorption de Kubelka-Munk de ladite plaque à un endothélium aortique normal est d'au moins 1,5:1, et de préférence au moins 2:1, la durée d'émission étant inférieure au temps de relaxation thermique du volume de tissu défini par le diamètre du rayon laser sur le tissu et une profondeur à laquelle 67% de la lumière incidente dudit laser sont absorbés, l'intensité du rayon étant suffisamment forte pour, par le choix d'une longueur d'onde et d'une durée d'émission définies, entraîner l'échauffement de la plaque ciblée à une température suffisante pour éliminer par brûlage une partie importante de ladite plaque sans causer de dommage inacceptable aux tissus sains environnants.

9.  Utilisation du $\beta$-carotène pour la fabrication d'un médicament devant être administré à un patient selon le traitement défini dans la Revendication 8.

10. Utilisation d'une denrée alimentaire contenant un caroténoïde pour la fabrication d'un médicament devant être administré à un patient selon le traitement défini dans la Revendication 8.

11. Utilisation d'un aliment naturel contenant un caroténoïde pour la préparation d'un produit devant être ingéré par un patient dans le traitement visant l'ablation de sa plaque athérosclérotique, traitement au cours duquel, après une telle ingestion, la plaque est exposée à une lumière laser émise selon la Revendication 8.

FIG 1

BECKMAN 5270
UV – VIS
SPECTROPHOTOMETER  14

HP 9825A
CALCULATOR  16

12 FIBEROPTIC BUNDLE

REFERENCE BEAM

10

18 COLLIMATING LENS

LEAD-SULFIDE CELL  26

FIBEROPTIC BUNDLE

21

20 QUARTZ PLATES

SAMPLE BEAM  24

SPECIMEN PLACED HERE FOR TRANSMITTANCE

SPECIMEN PLACED HERE FOR REMITTANCE

PHOTOMULTIPLIER TUBE (HAMAMUTSU)
22

3" DIA. INTEGRATING SPHERE IN A LIGHT TIGHT BOX

FIG 2

100

FLASHLAMP PUMPED DYE LASER

1cm

20cm FOCAL LENGTH

AORTA

2-3mm

120

TRANSLATING MOUNT

21cm

PLAQUE TO NORMAL RATIO OF
KUBELKA-MUNK
ABSORPTION COEFFICIENTS

FIG 3

EP 0 200 390 B1